(19) Europäisches Patentamt
European Patent Office
Office européen des brevets

(11) Numéro de publication: **0 013 844**
**B1**

(12) **FASCICULE DE BREVET EUROPEEN**

(45) Date de publication du fascicule de brevet: **04.11.81**

(21) Numéro de dépôt: **79400906.8**

(22) Date de dépôt: **23.11.79**

(51) Int. Cl.[3]: **C 07 C 69/732**, C 07 C 69/734, C 07 C 67/343, C 07 C 67/08, C 07 C 69/16, C 07 C 69/747

(54) **Dérivés substitués de l'acide pentèn-2-yle malonique; procédé pour leur préparation et leur application dans la synthèse d'un précurseur de l'acide chrysanthémique.**

(30) Priorité: **13.12.78 FR 7835047**

(43) Date de publication de la demande:
**06.08.80 Bulletin 80/16**

(45) Mention de la délivrance du brevet:
**04.11.81 Bulletin 81/44**

(84) Etats Contractants Désignés:
**CH DE GB NL**

(56) Documents cités:
**Neant**

(73) Titulaire: **ROUSSEL-UCLAF**
**102, route de Noisy Boîte postale no.9**
**F-93230 Romainville (FR)**

(72) Inventeur: **Ficini, Jacqueline**
**15, Rue Buffon**
**F-75005 Paris (FR)**
Inventeur: **Genet, Jean-Pierre**
**12, Rue Jacquemin**
**F-92260 Fontenay aux Roses (FR)**

(74) Mandataire: **Niel, Michel et al,**
**boîte postale no 9 102, route de Noisy**
**F-93230 Romainville (FR)**

**EP 0 013 844 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Dérivés substitués de l'acide pentèn-2-yle malonique; procédé pour leur préparation et leur application dans la synthèse d'un précurseur de l'acide chrysanthémique

La présente invention concerne de nouveaux dérivés substitués du pentèn-2-yle, leur procédé de préparation et leur application dans la synthèse d'un précurseur de l'acide chrysanthémique.

La présente invention a pour objet les composés de formule I:

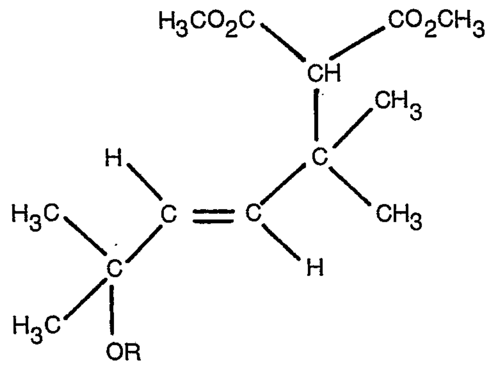

(I).

de structure trans, dans laquelle R représente un atome d'hydrogène ou un radical acétyle.

L'invention a plus particulièrement pour objet, les composés de formule I dont les noms suivent:

—le (4-hydroxy 1,1,4-triméthyl pent-2-ényl)propanedioate de méthyle (trans),

—le (4-acétyloxy 1,1,4-triméthyl pent-2-ényl)propanedioate de méthyle (trans).

L'invention a également pour objet un procédé de préparation des composés de formule I, telle que définie précédemment, caractérisé en ce que l'on traite le composé de formule II:

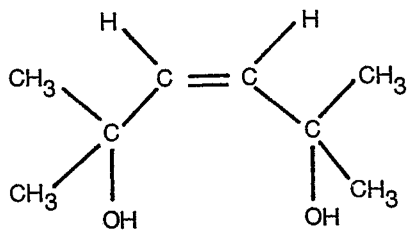

(II)

de structure cis, par un agent d'acétylation, pour obtenir le monoacétate correspondant de structure cis, de formule III:

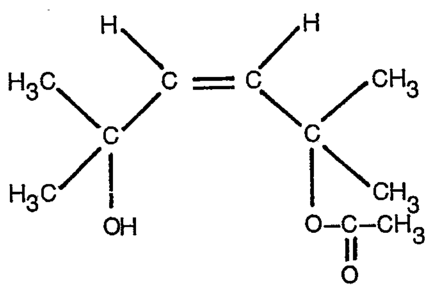

(III)

que l'on fait réagir avec le dérivé sodé malonique de formule IV:

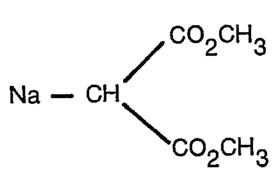

(IV)

en présence d'un complexe du palladium (o) au sein d'un solvant organique, pour obtenir le composé de formule IA:

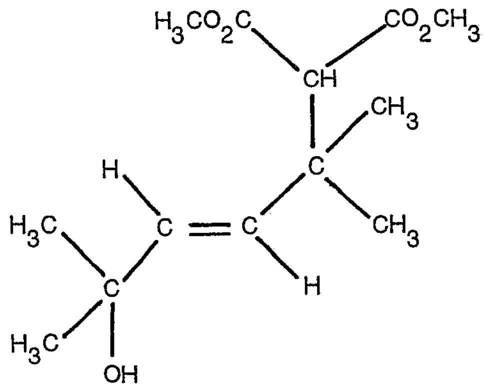

(IA)

de structure trans, correspondant à un composé de formule I, dans laquelle R représente un atome d'hydrogène, composé de formule IA que l'on traite, le cas échéant, par un agent d'acétylation, pour obtenir le composé de formule IB:

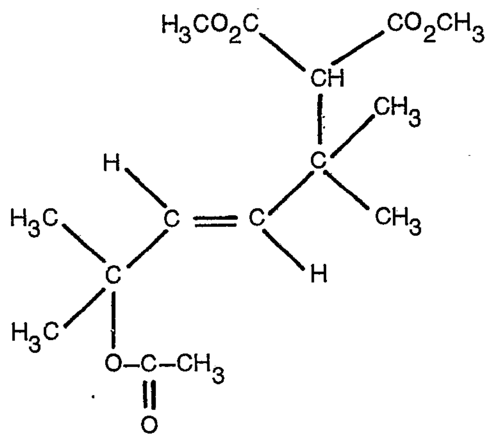

(IB)

de structure trans, correspondant à un composé de formule I, dans laquelle R représente un radical acétyle.

Dans des conditions préférentielles de mise en oeuvre de l'invention, le procédé de préparation décrit ci-dessus est caractérisé en ce que:

a) la monoacétylation du composé de formule II est effectuée par l'anhydride acétique en présence de triéthylamine et d'une quantité calalytique de 4-(N,N-diméthylamino)pyridine;

b) le complexe du palladium (o) utilisé dans la réaction du composé de formule III avec le composé de formule IV est le tétrakistriphényl phosphine palladium;

c) l'acétylation du composé de formule IA est effectuée dans les mêmes conditions que celles du composé de formule II.

Le procédé de l'invention permet de préparer les composés de formule I avec d'excellents rendements et à partir d'un matière première de structure chimique simple et d'un faible coût.

L'utilisation de dérivés du palladium dans la substitution des acétates allyliques est une méthode qui est connue seulement dans le cas d'acétates monofonctionnels (J. Org. Chem. *41*, 3216 (1976). L'intérêt du procédé, objet de l'invention, réside essentiellement dans le fait que, d'une part, la fonction acétate n'est éliminée que grâce à la présence du palladium, l'unité malonique étant régiosélectivement introduite et que, d'autre part, le réaction est stéréosélective et permet l'isomérisation de la structure cis en la structure trans recherchée. L'utilisation du dérivé du palladium complexant la double liaison permet en effet l'obtention univoque de la structure trans.

Les composés de formule I sont des composés utiles comme intermédiaires dans la synthèse de l'acide chrysanthémique et notamment de l'un de ses précurseurs possibles, à savoir le composé de formule V:

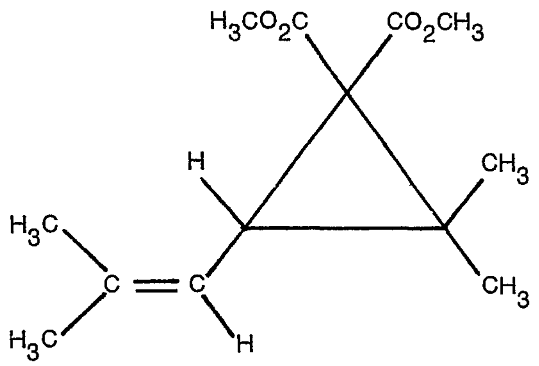

(V)

Ce composé, décrit dans la littérature (Bull. soc. Chim. Japan *50* (10) 2825-6 1977) conduit en effet, par action du chlorure de lithium dans le diméthylsulfoxyde en présence d'eau, selon une technique décrite dans J.Org. Chem. *43* 138 (1978), au monoester méthylique correspondant qui, par épimérisation en milieu basique forte, comme par exemple en présence d'éthylate de sodium dans l'éthanol, selon une technique décrite dans Bull. Soc. Chim. de France 3499 (1966), conduit au trans chrysanthémate de méthyle puis, par saponification, à l'acide trans chrysanthémique.

L'invention a ainsi également pour objet l'application du composé de formule I, telle que définie précédemment, dans laquelle R représente un radical acétyl, à la préparation du composé de formule V:

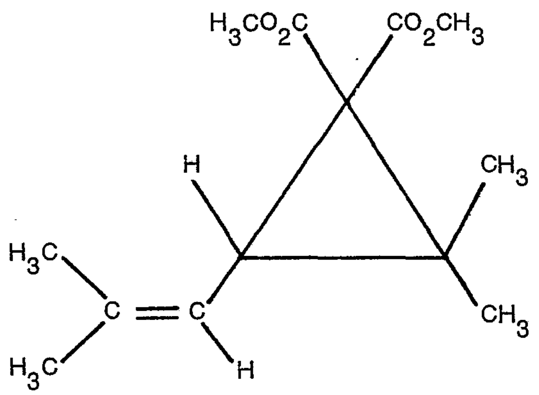

(V)

caractérisée en ce que l'on fait réagir ledit composé de formule I:

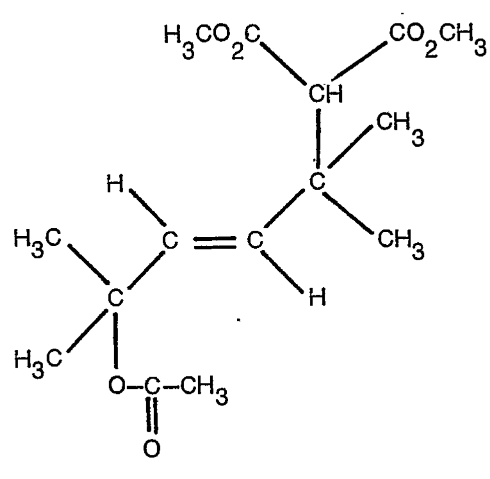

(I)

avec un hydrure alcalin au sein d'un solvant organique puis avec un complexe du palladium (o) pour obtenir le composé de formule V attendu.

Dans des conditions préférentielles d'exécution du procédé, objet de l'application ci-dessus, on fait réagir le composé de formule I avec de l'hydrure de sodium au sein d'un mélange de térahydrofuranne et d'hexaméthylphosphototriamide puis verse le mélange dans une solution de tétrakistriphenylphosphine palladium dans le même mélange de solvants.

Il va de soi que l'invention s'étend à un procédé de préparation du composé de formule V au départ du composé de formule I dans laquelle R représente un atome d'hydrogène, c'est-à-dire du composé de formule IA, procédé selon lequel on prépare dans un premier temps un produit de formule IB selon le procédé décrit précédemment, puis fait réagir ce composé de formule IB avec un hydrure

alcalin puis avec le complexe du palladium (o) selon le procédé décrit précedemment.

L'intérêt de l'utilisation du dérivé du palladium dans la réaction de cyclisation ci-dessus, provient du fait que la réaction qui est régiosélective est accélérée par la présence du palladium. Celle-ci conduit en effet dans de bonnes conditions, par l'attaque du pôle le moins encombré du complexe formé, à la structure attendue.

En définitive, l'invention permet d'accéder à la structure chrysanthémique trans, et notamment de préparer le trans chrysanthémate de méthyle, selon une voie nouvelle, en un nombre restreint de stades et au départ d'un dérivé de l'hex-2-ène facilement accessible.

Les composés de formule I, puis le composé de formule V sont obtenus dans des conditions parfaitement reproductibles et avec un rendement global intéressant. Ce composé de formule V, ainsi qu'il est décrite dans les publications mentionnées plus haut, peut ensuite être transformé en trans chrysanthémate de méthyle avec un excellent rendement, en deux stades.

Le composé de départ de formule II est un composé connu et décrit, par example dans J.Org. Chem. *27* 2398 (1962) ou CA. *59* 2675a ou *85* 76937 m (1976).

Les exemples suivants illustrent l'invention sans toutefois la limiter.

### Exemple 1

(4-hydroxy 1,1,4-triméthyl pent-2-ènyl) propanedioate de méthyle trans.

*Strade A:* Monoacétate de 2,5-dimethyl hex-3-ène 2,5-diol (cis)

On dissout 10 g de 2,5-dimethyl hex-3-ène 2,5-diol (cis) dans 10 cm3 de chlorure de méthylène, ajoute 30 cm3 de triéthylamine et 0,840 g de 4-(N,N-diméthylamino) pyridine puis ajoute en 4 heures sous agitation à température ambiante, 7,1 g d'anhydride acétique. On ajoute ensuite du chlorure de méthylène, lave avec une solution aqueuse d'acide chlorhydrique à 5% puis à l'eau, sèche et évapore le solvant sous pression réduite. On obtient 9,6 g de produit attendu que l'on purifie par chromatographie sur silice en éluant au mélange éther-pentane (1-25) puis par distillation. On obtient 7,8 g de produit pur. Eb. $_{0,1}$ = 60°C.

*Stade B:* 4-hydroxy 1,1,4-triméthyl pent-2-ènyl propanedioate de méthyle trans.

A 4,86 g du produit obtenu au stade A, on ajoute à 70°C, une solution de dérivé sodé du malonate de méthyle préparée à partir de 0,48 g d'hydrure de sodium à 50% dans l'huile, 1,5 cm3 de malonate de méthyle et 15 cm3 de tétrehydrofuranne anhydre. On ajoute ensuite 0,6 g de tétrakistriphenyl phosphine palladium et mainteint sous agitation à 70°C pendant 16 heures.
On refroidit, verse dans l'eau, extrait à l'éther, sèche la phase organique et évapore à sec sous pression réduite. On distille le résidu ($Eb._{0,01}$ = 120—130°C) et obtient 2,2 g de produit attendu.

Le 2,5-diméthyl hex-3-ène 2,5-diol utilisé au départ de l'exemple 1 peut être préparé de la manière suivante:

On dissout 24 g de 2,5-diméthyl hex-3-yne 2,5-diol (composé décrit par F.E. Ray et Coll. Am. Soc. *74* 1247 (1952) dans 200 cm3 de méthanol, ajoute 1,6 g de palladium sur carbonate de calcium à 5% et 0,6 g de quinoléine et agite le mélange sous hydrogène. A la fin de l'absorption d'hydrogène, on filtre le catalyseur et évapore le solvant sous pression réduite. On obtient 24,2 g de produit attendu (F = 64°C) utilisé tel que pour le stade suivant.

### Exemple 2

(4-acétyloxy 1,1,4-triméthyl pent-2-ényl) propanedioate de méthyle trans.

On dissout 2 g de produit obtenu à l'exemple 1 dans 6 cm3 de chlorure de méthylène et ajoute 2 cm3 d'anhydride acétique, 2 cm3 de triéthylamine et 0,084 g de 4-(N,N-diméthylamino) pyridine puis agite pendant 6 heures à température ambiente. On ajoute ensuite de chlorure de méthylène, lave avec une solution aqueuse d'acide chlorhydrique à 5% puis avec une solution aqueuse de bicarbonate de sodium et enfin à l'eau, sèche, évapore le solvant sous pression réduite, distille le résidu et obtient 1,98 g de produit attendu. $Eb_{0,1}$ = 100—110°C.

### Exemple 3

2,2-diméthyl 3-(2'-méthyl propényl) cyclopropane 1,1-dicarboxylate de méthyle.

On agite pendant 30 minutes à température ambiante un mélange de 0,3 g de (4-acétyloxy 1,1,4-triméthyl pent-2-ènyl) propanedioate de méthyle trans, obtenu à l'exemple 2, 0,8 cm3 de tétrahydrofuranne, 0,4 cm3 d'hexaméthyl phosphorotriamide et 0,048 g d'hydrure de sodium à 60% dans l'huile. On introduit ensuite le mélange à 70°C sous agitation en 30 minutes dans une solution de 0,16 g de tétrakistriphényl phosphine palladium dans 0,4 cm3 d'hexaméthyl phosphorotriamide et 0,8 cm3 de tétrahydrofuranne, agite pendant une heure 30 minutes, verse dans l'eau, extrait à l'éther, sèche la phase organique, évapore le solvant, chromatographie le résidu sur silice en éluant au mélange éther-pentane (1—4) et obtient 0,072 g de produit attendu.

**Revendications**

1. Les composés de formule:

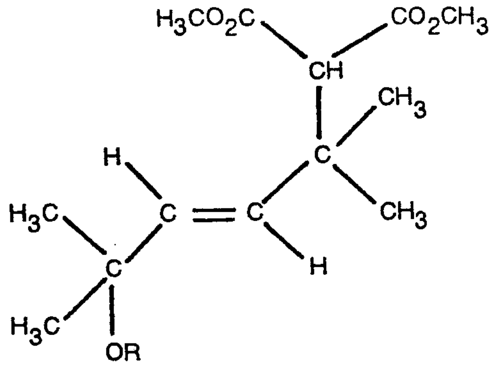

(I)

de structure trans, dans laquelle R représente un atome d'hydrogène ou un radical acétyle.

2. Le (4-hydroxy 1,1,4-triméthyl pent-2-ényl)propanedioate de méthyle trans.

3. Le (4-acétyloxy 1,1,4-triméthyl pent-2-ényl)propanedioate de méthyle trans.

4. Procédé de préparation des composés de formule I, telle que définie à la revendication 1, caractérisé en ce que l'on traite le composé de formule II:

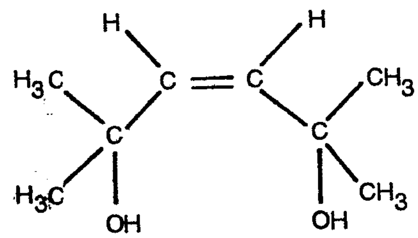

(II)

de structure cis, par un agent d'acétylation, pour obtenir le monoacétate correspondant de structure cis, de formule III:

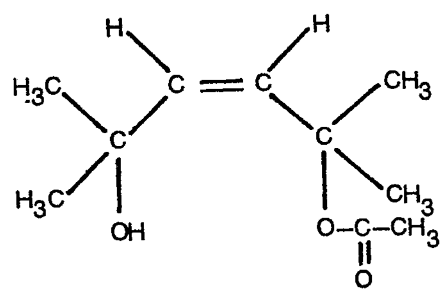

(III)

que l'on fait réagir avec le dérivé sodé malonique de formule IV:

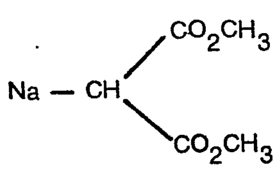

(IV)

en présence d'un complexe du palladium (o) au sein d'un solvant organique, pour obtenir le composé de formule IA:

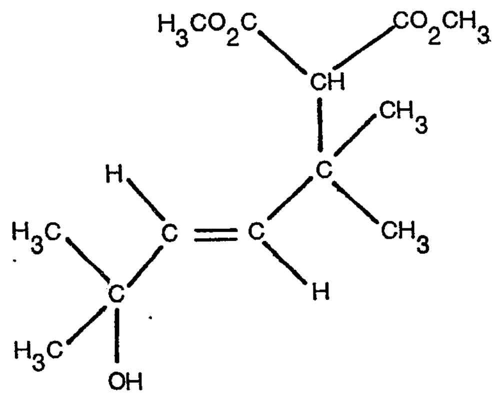

(IA)

de structure trans, correspondant à un composé de formule I, dans laquelle R représénte un atome d'hydrogène composé de formule IA que l'on traite, le cas échéant, par un agent d'acétylation, pour obtenir le composé de formule IB:

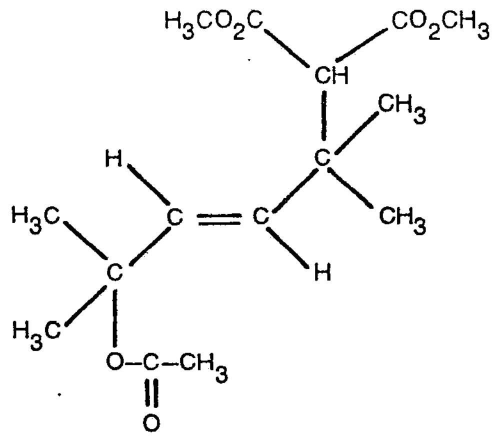

(IB)

de structure trans, correspondant à un composé de formule I, dans laquelle R représente un radical acétyle.

5. Procédé selon la revendication 4, caractérisé en ce que:

a) la monoacétylation du composé de formule II est effectuée par l'anhydride acétique en présence de triéthylamine et d'une quantité catalytique de 4-(N,N-diméthylamino) pyridine;

b) le complexe du palladium (o) utilisé dans la réaction du composé de formule III avec le composé de formule IV est le tétrakistriphényl phosphine palladium;

c) l'acétylation du composé de formule IA est effectuée dans les mêmes conditions que celles du composé de formule II.

6. Application du composé de formule I, telle que définie, à la revendication 1, dans laquelle R représente un radical acétyl, à la préparation du composé de formule V:

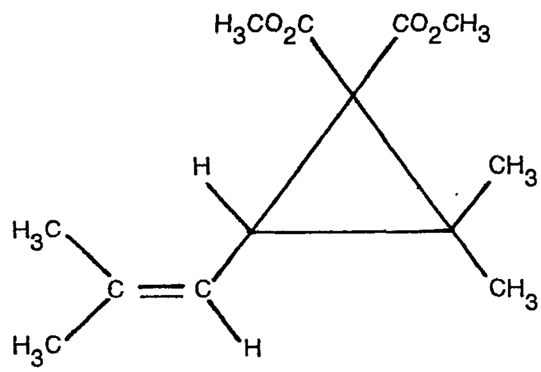

(V)

caractérisée en ce que l'on fait réagir ledit composé de formule I:

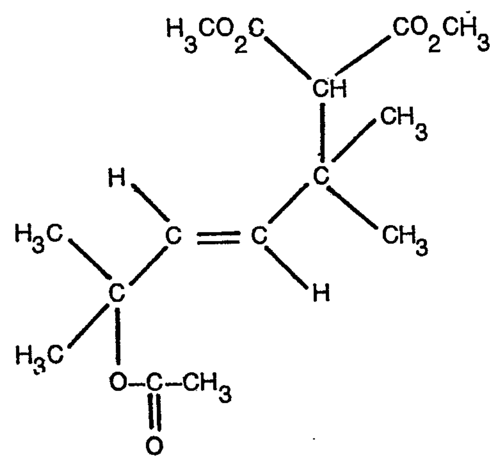

(I)

avec un hydrure alcalin au sein d'un solvant organique puis avec un complexe du palladium (o) pour obtenir le composé de formule V attendu.

7. Procédé selon la revendication 6, caractérisé en ce que l'on fait réagir le composé de formule I avec de l'hydrure de sodium au sein d'un mélange de tétrahydrofuranne et d'hexaméthyl phosphotriamide puis verse le mélange dans une solution de tétrakistriphényl phosphine palladium dans le même mélange de solvants.

**Patentansprüche**

1. Verbindungen der Formel

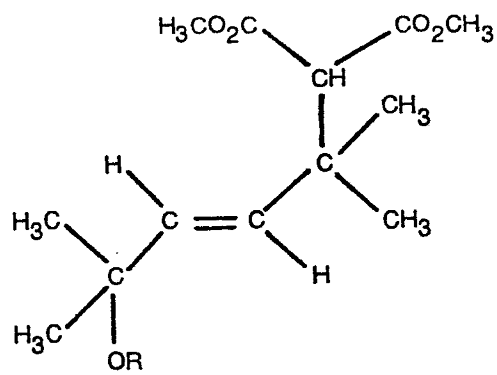

(I)

mit trans-Struktur, worin R ein Wasserstoffatom oder einen Acetylrest bedeutet.

2. Trans-(4-hydroxy-1,1,4-trimethyl-pent-2-enyl)-propandisäuremethylester.

3. Trans-(4-acetyloxy-1,1,4-trimethyl-pent-2-enyl)-propandisäuremethylester.

4. Verfahren zur Herstellung der Verbindungen der Formel I, gemäß Anspruch 1, dadurch gekennzeichnet, daß man die Verbindung der Formel II

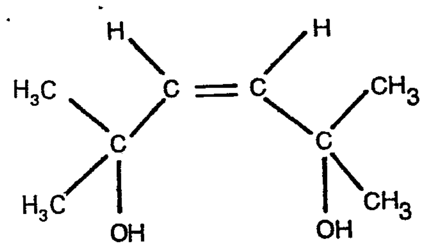

(II)

mit cis-Struktur mit einem Acetylierungsmittel behandelt, um das entsprechende Monoacetat mit cis-Struktur der Formel III

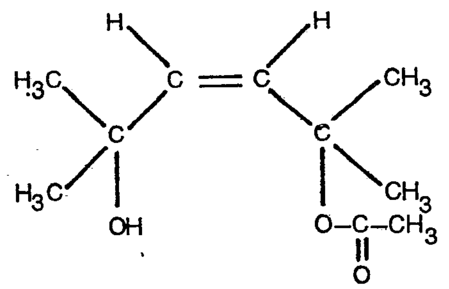

(III)

zu erhalten, das man mit dem Natriumderivat der Malonsäure der formel IV

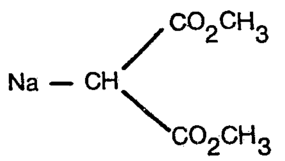

(IV)

in Gegenwart eines Komplexes von Palladium (O) in dem Medium eines organischen Lösungsmittels umsetzt, um die Verbindung der Formel IA

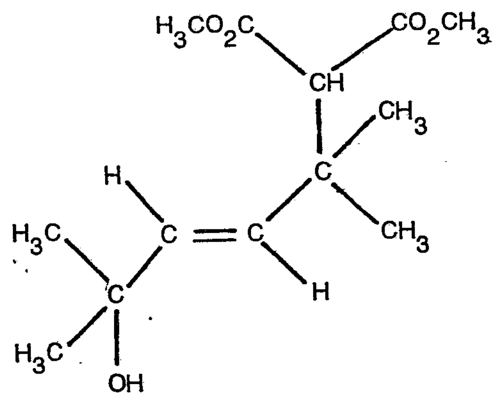

(IA)

mit trans-Struktur, entsprechend einer Verbindung der Formel I, worin R ein Wasserstoffatom bedeutet, zu erhalten, welche Verbindung der Formel IA man gegebenenfalls mit einem Acetylierungsmittel behandelt, um die Verbindung der Formel IB

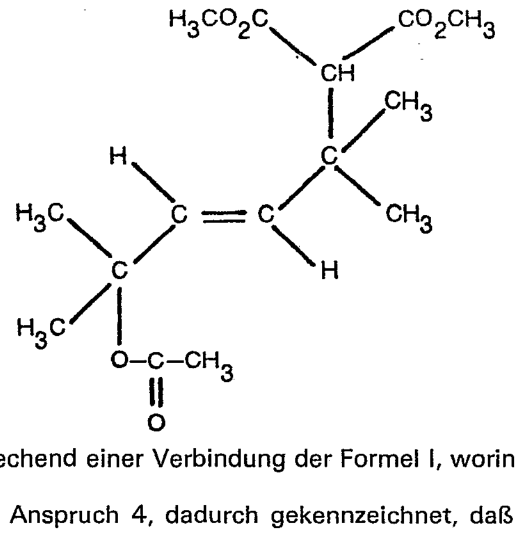

(IB)

mit trans-Struktur, entsprechend einer Verbindung der Formel I, worin R einen Acetylrest bedeutet, zu erhalten.

5. Verfahren gemäß Anspruch 4, dadurch gekennzeichnet, daß

a) die Monoacetylierung der Verbindung II mit Essigsäureanhybrid in Gegenwart von Triäthylamin und einer katalytischen Menge von 4-(N,N-Dimethylamino)-pyridin durchgeführt wird,

b) der Komplex des Palladiums (O), der bei der Umsetzung der Verbindung der Formel III mit der Verbindung der Formel IV verwendet wird, Tetrakistriphenylphosphinpalladium ist.

c) die Acetylierung der Formel IA unter den gleichen Bedingungen durchgeführt wird, wie denjenigen für die Verbindung der Formel II.

6. Verwendung der Verbindung der Formel I gemäß Anspruch 1 worin R einen Acetylrest bedeutet, zur Herstellung der Verbindung der Formel V

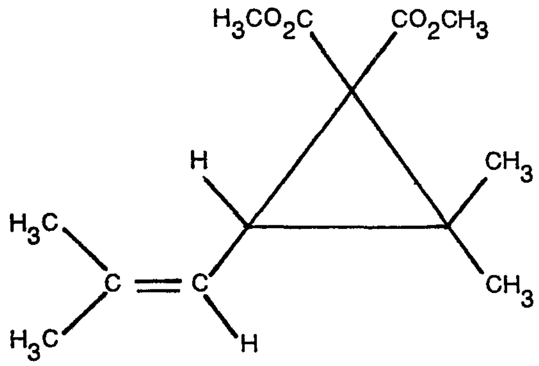

(V)

dadurch gekennzeichnet, daß man die Verbindung der Formel I

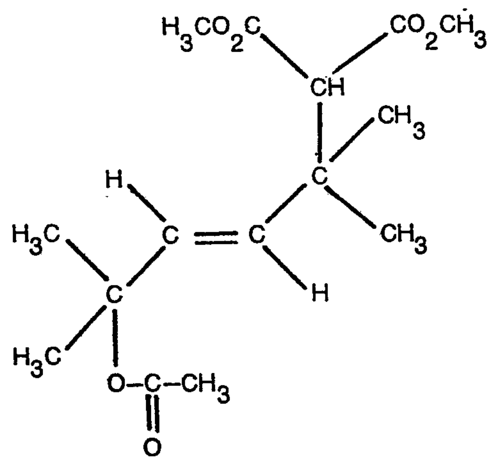

(I)

mit einem Alkalihydrid in dem Medium eines organischen Lösungsmittels und danach mit einem Komplex von Palladium (O) umsetzt, um gewünschte Verbindung der Formel V zu erhalten.

7. Verfahren gemäß Anspruch 6, dadurch gekennzeichnet, daß man die Verbindung der Formel I mit Natriumhydrid in dem Medium eines Gemisches von Tetrahydrofuran und Hexamethylphosphortrisamid umsetzt und danach das Gemisch in eine Lösung von Tetrakistriphenylphosphinpalladium in dem gleichen Lösungsmittelgemisch gießt.

**Claims**

1. The compounds of formula:

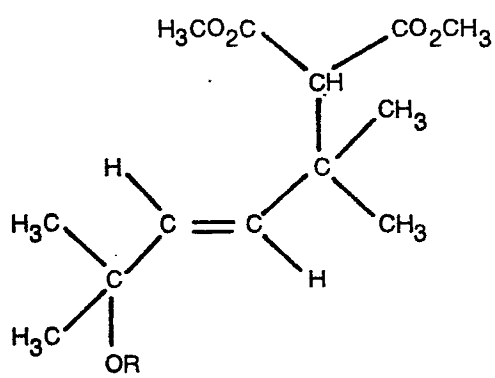

(I)

of trans structure, in which R represents a hydrogen atom or an acetyl radical.

2. Trans methyl (4-hydroxy 1,1,4-trimethyl pent-2-enyl) propanedioate.

3. Trans methyl (4-acetyloxy 1,1,4-trimethyl pent-2-enyl) propanedioate.

4. Process for preparing the compounds of formula I as defined in claim 1, characterised in that the compound of formula II:

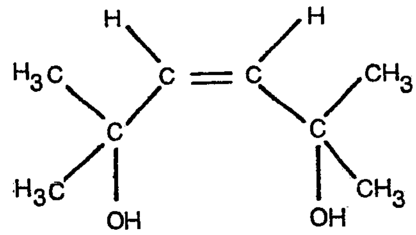

(II)

of cis structure, is treated with an acetylation agent to obtain the corresponding monoacetate of cis structure, of formula III:

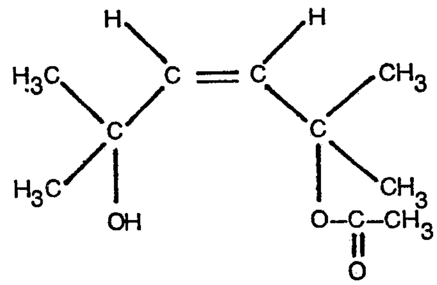

(III)

which is reacted with the sodium derivative of malonic acid of formula IV:

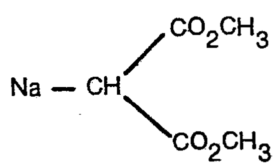

(IV)

in the presence of a complex of palladium (o) in an organic solvent, to obtain the compound of formula IA:

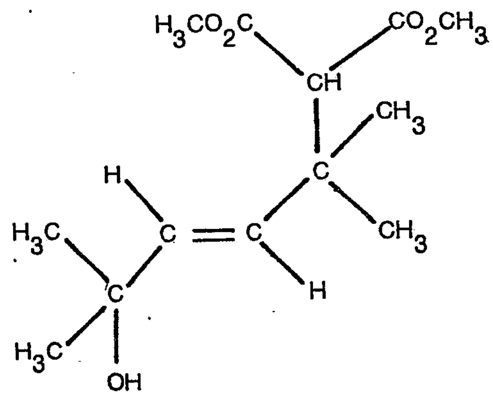

(IA)

of trans structure, corresponding to a compound of formula I, in which R represents a hydrogen atom, which compound of formula IA is treated, if applicable, with an acetylation agent, to obtain the compound of formula IB:

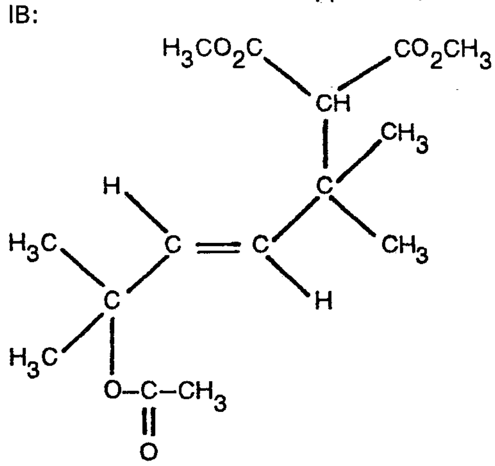

(IB)

of trans structure, corresponding to a compound of formula I, in which R represents an acetyl radical.

5. Process according to claim 4, characterised in that:

a) the monoacetylation of the compound of formula II is carried out with acetic anhydride in the presence of triethylamine and a catalytic amount of 4-(N,N-dimethylamino) pyridine;

b) the complex of palladium (o) used in the reaction of the compound of formula III with the compound of formula IV is tetrakistriphenyl phosphine palladium;

c) the acetylation of the compound of formula IA is carried out under the same conditions as those of the compound of formula II.

6. Use of the compound of formula I as defined in claim 1, in which R represents an acetyl radical, for the preparation of the compond of formula V:

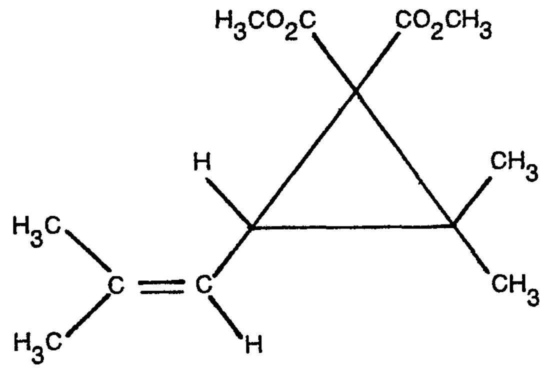

(V)

characterised in that the said compound of formula I:

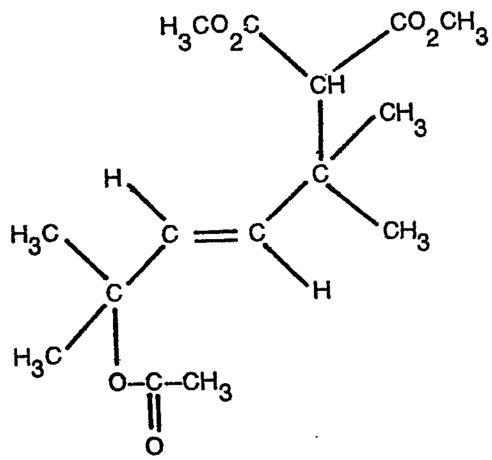

(I)

is reacted with an alkali hydride in an organic solvent then with a complex of palladium (o) to obtain the expected compound of formula V.

7. Process according to claim 6, characterised in that the compound of formula I is reacted with sodium hydride in a mixture of tetrahydrofuran and of hexamethylphosphotriamide, then the mixture is poured into a solution of tetrakistriphenyl phosphine palladium in the same mixture of solvents.